# EUROPÄISCHE PATENTSCHRIFT

(11) **EP 2 991 664 B1**
(45) Veröffentlichungstag und Bekanntmachung des Hinweises auf die Patenterteilung: **04.09.2019**
(21) Anmeldenummer: 14730065.1
(22) Anmeldetag: 17.04.2014
(51) Int. Cl.: A61K 38/10, A61K 38/12, A61K 38/16, A61K 9/06, A61K 9/20, A61P 25/28, A61P 31/18, C07K 14/47

(54) **AGENZIEN ZUR PRÄVENTION UND THERAPIE VON FOLGEERKRANKUNGEN VON HIV UND ANDERER VIRALER INFEKTIONEN**
AGENTS FOR THE PROPHYLAXIS AND TREATMENT OF SECONDARY DISEASES OF HIV AND OTHER VIRAL INFECTIONS
AGENTS DE PRÉVENTION ET DE TRAITEMENT DES MALADIES SECONDAIRES DES INFECTIONS PAR LE VIH ET D'AUTRES INFECTION VIRALES

(30) Priorität: 30.04.2013 DE 102013007405; 26.09.2013 DE 102013016002; 12.03.2014 DE 102014003262
(43) Veröffentlichungstag der Anmeldung: 09.03.2016
(73) Patentinhaber: Forschungszentrum Jülich GmbH, 52425 Jülich (DE)
(72) Erfinder: FUNKE, Susanne, Aileen, 96242 Sonnefeld (DE); WILLBOLD, Dieter, 52428 Jülich (DE); SCHAAL, Heiner, 50735 Köln (DE); WIDERA, Marek, 45147 Essen (DE)
(74) Vertreter: Fitzner, Uwe
(86) Internationale Anmeldenummer: PCT/DE2014/000203
(87) Internationale Veröffentlichungsnummer: WO 2014/177127

(56) Entgegenhaltungen:
- EP-A1- 2 179 722
- WO-A2-02/081505
- WO-A2-2005/060352
- WO-A2-2010/041252
- WO-A2-2011/133929
- DE-A1-102012 102 998
- DE-A1-102012 102 999
- US-A1- 2007 231 331
- US-A1- 2010 204 085
- SIEVERS STUART A ET AL: "Structure-based design of non-natural amino-acid inhibitors of amyloid fibril formation", NATURE, NATURE PUBLISHING GROUP, UNITED KINGDOM, Bd. 475, Nr. 7354, 1. Juli 2011 (2011-07-01), Seiten 96-100, XP001526382, ISSN: 0028-0836, DOI: 10.1038/NATURE10154 in der Anmeldung erwähnt
- CHRISTINA C. CAPULE ET AL: "Oligovalent Amyloid-Binding Agents Reduce SEVI-Mediated Enhancement of HIV-1 Infection", JOURNAL OF THE AMERICAN CHEMICAL SOCIETY, Bd. 134, Nr. 2, 18. Januar 2012 (2012-01-18), Seiten 905-908, XP055092256, ISSN: 0002-7863, DOI: 10.1021/ja210931b in der Anmeldung erwähnt
- W. M. Wojtowicz ET AL: "Stimulation of Enveloped Virus Infection by -Amyloid Fibrils", Journal of Biological Chemistry, vol. 277, no. 38, 15 July 2002 (2002-07-15), pages 35019-35024, XP055138231, ISSN: 0021-9258, DOI: 10.1074/jbc.M203518200

## Beschreibung

Die Erfindung bezieht sich auf das Gebiet der Prävention und Therapie von HIV- und anderen viralen Infektionen.

### Stand der Technik

Das Humane Immundefizienz-Virus, abgekürzt HIV, gehört zur Familie der Retroviren und zur Gattung der Lentiviren. Eine unbehandelte HIV-Infektion führt in der Regel nach einer unterschiedlich langen, meist mehrjährigen Inkubationszeit zu AIDS (engl. acquired immunodeficiency syndrome, erworbenes Immundefizienzsyndrom). Die Verbreitung von HIV hat sich in den letzten 30 Jahren zu einer Pandemie entwickelt, die nach Schätzungen der Organisation UNAIDS bisher etwa 28 Millionen Leben gefordert hat. Ende 2010 waren geschätzt 34 Millionen Menschen weltweit mit HIV infiziert. Die Krankheit kann bisher - bis auf einen beschriebenen Einzelfall - nicht geheilt werden und erfordert eine lebenslange Therapie, bei der durch die Hemmung der HIV-Replikation infektionsbedingte Symptome unterdrückt werden.

Nach der Deutsch-Österreichischen Leitlinie zur antiretroviralen Therapie der HIV-Infektion ist das Ziel antiretroviraler Therapien (ART), durch die Hemmung der HIV-Replikation infektionsbedingte Symptome zu unterdrücken und die Krankheitsprogression zu vermindern. Die Prognose HIV-infizierter Patienten hat sich durch die HAART (Hochaktive antiretrovirale Therapie) erheblich verbessert. Heute erreichen die Patienten ein verhältnismäßig hohes Lebensalter. Die Therapie ist allerdings lebenslang erforderlich, da die virale Infektion in der Regel nur unterdrückt, nicht aber ausgeheilt wird. Die körperliche und psychische Belastung durch die dauerhafte Medikamenteneinnahme und durch häufiger auftretenden Infektionen ist hoch, ebenso die Kosten der jahrelangen HAART. Durch die HAART wird das Ansteckungsrisiko für HIV durch erniedrigten Virentiter zwar verringert. Die Ansteckung kann aber nicht vermieden werden. Eine Verhinderung der Ansteckung ist daher erstrebenswert.

Das HI-Virus wird durch Kontakt mit Körperflüssigkeiten wie Blut, Sperma, Vaginalsekret und Muttermilch übertragen. Die häufigsten Infektionswege sind Intimverkehr ohne Verwendung von Kondomen und die Benutzung unsteriler Spritzen beim intravenösen Drogenkonsum. Trotz der großen Bedeutung von Spermaflüssigkeit bei der sexuellen Übertragung von HIV ist bisher noch wenig über die Übertragungswege bekannt.

2007 wurde über in der Samenflüssigkeit enthaltende amyloidogene Proteine, genannt SEVI (semen derived enhancer of virus infection), berichtet, die die HIV-Infektion verschiedener Zelltypen in Zellkultur und Gewebemodellen verstärken konnten. Diese spielen möglicherweise eine wichtige Rolle bei der sexuellen Übertragung von HIV, da sie den Kontakt zwischen Virus und Wirtszelle erleichtern können (Münch et al., Cell 131, 1059-1071).

Im Umkehrschluss sollte durch die Verhinderung der SEVI-HIV-Zell-Interaktion die sexuelle Übertragung von HIV verhindert werden können. Erste experimentelle Ansätze zur Verhinderung der SEVI-vermittelten HIV-Infektion durch den Einsatz von Amyloid-bindenden Substanzen in Zellkultur wurden bereits vorgestellt (Capule et al., JACS 134, 905-908). Die Entwicklung ist noch nicht weit fortgeschritten.

Aus der Druckschrift Sievers et al. (Nature (2011). 475, 96-100) sind D-Peptide bekannt, die an ein Hexapeptid VQIVYK (Reste 306-311) des Tau-Proteins, welches mit der Alzheimerschen Demenz assoziiert ist, binden. Aus dieser Druckschrift ist auch ein L-Peptid bekannt, das ebenfalls an ein Hexapeptid GGVLVN des ²⁴⁸PAP²⁸⁶ -Protein von menschlichem Samen bindet. Die Fibrillenbildung konnte jeweils spezifisch mit dem D-Peptid für das Tau-Protein und mit dem L-Peptid für das PAP-Protein verhindert werden.

Wojtowicz et al. berichteten im Jahr 2002 darüber, dass synthetische, amyloide Fibrillen aus Aβ₁₋₄₀ und Aβ₁₋₄₂ die Infektiosität von dem HIV-1 Virus steigern können (The Journal of Biological Chemistry (2002). 277, 35019-35024). Die Aβ-Fibrillen verursachen eine 5 bis 20-fach erhöhte Infektionseffizienz von Reporterzellen, die den HIV-1 Rezeptor CD4 exprimieren.

WO 2005/060352 A2 beschreibt einen wässerigen Extrakt, der aus einer Zimtrinde gewonnen wird und eine antivirale Aktivität aufweist.

WO 2010/041252 A2 offenbart die Verwendung eines Zimtextrakts bei der Behandlung Amyloid-assoziierter Erkrankungen und Störungen.

US 2007/231331 A1 betrifft neurodegenerative Erkrankungen und eine Gruppe von presenilin-, G-Protein- und c-Src-bindenden Polypeptiden und die Anwendung bei der Alzheimer-Krankheit.

WO 02/081505 A2 offenbart Peptide, die mit einer hohen Bindungsaffinität an das Peptid beta-Amyloid binden, wie z.B. das Peptid D3, und die Verwendung dieser Peptide für die Diagnose und Therapie der Alzheimer-Krankheit.

DE 10 2012 102 999 A1 bezieht sich auf ein Verfahren zur Behandlung von Blut, Blutprodukten und/oder Organen unter in vitro und ex vivo-Bedingungen, bei dem Amyloid-beta-Oligomere unter Verwendung spezifischer Peptide entfernt werden.

DE 10 2012 102 998 A1 offenbart Polymere, die mindestens zwei Substanzen (Monomere) umfassen, die an Amyloid-beta-Oligomere binden.

EP 2 179 722 A1 stellt eine pharmazeutische Zusammensetzung zur topischen Verabreichung zur Verhinderung der sexuellen Übertragung einer HIV-Infektion bereit, wobei die Zusammensetzung eine Verbindung aus der Klasse der Catechine umfasst.

US 2010/204085 A1 stellt hemmende peptidische Verbindungen bereit, die in pharmazeutischen Zusammensetzungen und Verfahren zur Behandlung von mit Polypeptidaggregation verbundenen Krankheiten verwendet werden können.

WO 2011/133929 A2 stellt Verfahren zur Verfügung, um eine sexuell übertragbare Infektion einer Person zu behandeln oder zu verhindern. Die Verfahren umfassen die Verabreichung einer Substanz die SEVI (semen-derived enhancer of viral infection) bindet.Aufgabe der Erfindung

Aufgabe der Erfindung ist es, Agenzien zur Prävention und Therapie von HIV und anderen viralen Infektionen bereit zu stellen. Für die Verhinderung einer Infektion kommen besonders, aber nicht ausschließlich, SEVI-Fibrillen in Sperma als Zielmoleküle in Betracht. Bei bereits Infizierten Personen soll insbesondere, aber nicht ausschließlich, die Entstehung von HIV-assoziierten neurologischen Schädigungen des zentralen Nervensystems (z.B.: "HIV-assoziierte Demenz", HAD) verhindert werden oder deren Progression verlangsamt werden, in dem die Zahl der im ZNS mit zunehmendem Lebensalter immer häufiger vorkommenden Amyloid-Fibrillen (bestehend z.B. aus Aβ) reduziert wird und so die Infektionseffizienz von HIV möglichst stark verringert wird.

### Lösung der Aufgabe

Die Aufgabe der Erfindung wird gelöst durch ein Agenz, enthaltend mindestens eine Substanz, die dazu geeignet ist, die Bildung von amyloiden Fibrillen, die die Infektionseffizienz von HIV erhöhen, zu inhibieren oder diese zu zerstören, zur Anwendung in einem Verfahren für die Behandlung und / oder Prävention einer HIV-assoziierten Demenz (HAD), in dem amyloide Fibrillen, die die virale Infektionseffizienz erhöhen, in ihrer Bildung durch die Substanz verhindert werden oder diese zerstört werden, gekennzeichnet durch mindestens ein Peptid als Substanz mit einer Aminosäuresequenz ausgewählt aus der Gruppe bestehend aus SEQ ID NO: 1 und / oder SEQ ID NO: 2 und / oder SEQ ID NO: 3 und / oder SEQ ID NO: 4 und / oder SEQ ID NO: 5 und / oder SEQ ID NO: 6 und / oder SEQ ID NO: 7 und / oder SEQ ID NO: 8 und / oder SEQ ID NO: 9 und / oder SEQ ID NO: 10 und / oder SEQ ID NO: 11 und wobei mindestens ein Peptid im Wesentlichen oder ganz aus D-Aminosäuren besteht.

### Beschreibung der Erfindung

Die Aufgabe wird gelöst durch ein anspruchsgemäßes Agenz, das geeignet ist, die Bildung von amyloiden Fibrillen zu verhindern oder diese zu zerstören.

Das Agenz enthält mindestens eine Substanz, die dazu geeignet ist, die Bildung von amyloiden Fibrillen, die die Infektionseffizienz eines Virus erhöhen, zu inhibieren oder diese zu zerstören, zur Behandlung und / oder Prävention der jeweiligen viralen Infektion.

Geeignete Agenzien sind zum Beispiel Substanzen, die mindestens eine Aminosäuresequenz enthalten, die zur Prävention und Therapie der Alzheimerschen Demenz entwickelt bzw. erfunden wurde, in dem die damit assoziierten Amyloid-Fibrillen durch Peptide mit dieser Sequenz in ihrer Bildung verhindert werden oder diese zerstört werden. Diese dienen dann zur Behandlung und / oder Prävention von HIV und/oder anderer viraler Infektionen. Insbesondere dienen sie dazu, die weitere Ausbreitung der Infektion im Körper, z.B. im ZNS, zu verhindern oder diese zu behandeln.

Erfindungsgemäße Agenzien senken die durch amyloide Fibrillen (z. B. Aβ₁₋₄₂- oder SEVI-Fibrillen) gesteigerte Infektiösität von Viren vorzugsweise vollständig. Sie senken die durch Fibrillen gesteigerte Infektiösität von Viren, wie z. B. HIV, insbesondere um 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 oder 100%, wobei jeder Zwischenwert angenommen werden kann. Erfindungsgemäße Agenzien, nämlich die Peptide gemäß der SEQ ID NO: 1-11 senken die gesteigerte Infektiösität um mindestens 50%, vorzugsweise mindestens 60%, besonders bevorzugt um mindestens 70% und ganz besonders bevorzugt um mindestens 80% bzw. 90 oder mindestens 95%.

Es wurde erkannt, dass die bisherigen antiretroviralen Therapien von HIV Infektionen in der Regel schwere Nebenwirkungen haben. Wirkstoffe gegen die reverse Transkriptase bewirken nachteilig auch eine Beeinträchtigung anderer Lebensnotwendiger Moleküle z. B. die DNA Polymerase. Dadurch werden häufig teilende Zellen wie Haarfolikelzellen und Zellen der Blutbildung besonders betroffen (Anämien). Erfindungsgemäße Agenzien, nämlich die Peptide gemäß der SEQ ID NO: 1-11 haben keine Nebenwirkung auf die DNA Polymerase, bzw. auf die Zellen des Blutbildungssystems.

Im Vergleich hierzu, wird durch die erfindungsgemäßen Agenzien vorteilhaft bewirkt, dass die darin enthaltenden Peptide gemäß der SEQ ID NO: 1-11 nebenwirkungsfrei sind. Diese bewirken vorteilhaft, dass lediglich die amyloiden Fibrillen mit Infektionssteigernder Wirkung in ihrer Bildung verhindert oder zerstört werden, nicht aber die Monomere, aus denen Sie bestehen und die möglicherweise eine positive Funktion im Körper aufweisen.

Ein offenbartes Agenz, enthaltend mindestens eine Substanz, die dazu geeignet ist, die Bildung von insbesondere Aβ-Fibrillen zu inhibieren oder diese zu zerstören, wird zur Behandlung und / oder Prävention von HIV und / oder anderer viraler Infektionen sowie Folgeerkrankungen, wie z. B. HAD verwendet, in dem amyloide Fibrillen, die die virale Infektionseffizienz erhöhen, in ihrer Bildung verhindert werden oder diese zerstört werden.

Ein offenbartes Agenz, enthaltend mindestens ein Peptid mit einer Aminosäuresequenz, das zur Prävention und Therapie der Alzheimerschen Demenz assoziierten Fibrillen geeignet ist und / oder Homologe, Fragmente und Teile davon, wird zur Behandlung und / oder Prävention von HIV und / oder anderer viraler Infektionen sowie Folgeerkrankungen, wie z.B. HAD verwendet, in dem amyloide Fibrillen, die die virale Infektionseffizienz erhöhen, in ihrer Bildung verhindert werden oder diese zerstört werden.

In einer Ausgestaltung der Erfindung ist das erfindungsgemäße Peptid ein Peptid, enthaltend mindestens eine Aminosäuresequenz, welche an Amyloid-Beta-Spezies, z. B. an Aβ₁₋₄₂ bindet und bei der der freie C-terminus, also die C-terminale Carboxylguppe, derart modifiziert ist, dass der C-terminus keine negative Ladung trägt, sondern stattdessen neutral ist oder eine oder mehrere positive Ladungen aufweist. Unter die erfindungsgemäßen Peptide fallen auch solche Peptide, umfassend mindestens ein an eine Amyloid-Beta-Spezies bindendes Peptid, wobei das Peptid eine lineare Aminosäuresequenz aufweist, die es dazu befähigen, an A-Beta, z. B. an Aβ₁₋₄₂ zu binden, und diese Eigenschaft entweder erhalten bleibt oder verstärkt wird, in dem das Peptid durch kovalente Verknüpfung seiner beiden Enden in zyklisierter Form vorliegt.

Durch derartige erfindungsgemäße Peptide wird besonders vorteilhaft zusätzlich die Aufgabe gelöst, dass ein Peptid ohne negativen Ladungsüberschuss am C-terminus, bereitgestellt wird. Dadurch wird vorteilhaft bewirkt, dass dieses mit höherer Affinität an die Bestandteile von Amyloid-Fibrillen, wie z.B. SEVI oder Aβ, binden und diese zerstören kann, als ein Peptid, welches eine Carboxylgruppe am freien C-terminus aufweist.

In einer Ausgestaltung der Erfindung sind derartige erfindungsgemäßen Peptide im physiologischen Zustand, insbesondere bei pH 6-8, insbesondere 6,5-7,5, insbesondere bei pH 6,0, pH 6,1, pH 6,2, pH 6,3, pH 6,4, pH 6,5 pH 6,6, pH 6,7, pH 6,8, pH 6,9, pH 7,0, pH 7,1, pH 7,2, pH 7,3, pH 7,4, pH 7,5, pH 7,6, pH 7,7, pH 7,8, pH 7,9 bzw. pH 8,0 oder deren Zwischenwerte derart modifiziert, dass der C-terminus keine negative Ladung trägt, sondern stattdessen neutral ist oder eine oder mehrere positive Ladungen aufweist.

In einer weiteren Ausgestaltung der Erfindung ist das Peptid dadurch gekennzeichnet, dass am freien C-terminus an Stelle der Carboxylgruppe eine Säureamidgruppe vorliegt. Statt der Carboxylgruppe (-COOH-Gruppe) ist also eine Säureamidgruppe (-CONH₂-Gruppe) am C-terminus angeordnet. Das Peptid ist demnach besonders vorteilhaft am freien C-Terminus amidiert. Dadurch wird besonders vorteilhaft die weitere Aufgabe gelöst, dass ein Peptid ohne negativen Ladungsüberschuss vorliegt, welches affiner an das Zielmolekül, das heißt an A-Beta-Fibrillen und / oder SEVI assoziierte Fibrillen binden und diese zerstören kann und auf einfache Weise erhältlich ist.

In einer weiteren Ausgestaltung der Erfindung liegen folgende weitere Gruppen an Stelle der Carboxylgruppe vor: COH, COCl, COBr, CONH-alkyl-Rest, CONH-alkylAmin-Rest (positive Netto-Ladung) und so weiter, wobei man hierauf nicht beschränkt, sofern der technischen Lehre gefolgt wird, dass der C-terminus keine negative Ladung trägt, sondern stattdessen neutral ist oder eine oder mehrere positive Ladungen aufweist.

Offenbart werden Peptide enthaltend eine Aminosäuresequenz gemäß SEQ ID NO: 1 (D3), SEQ ID NO: 2 (RD2), SEQ ID NO: 3 (DB3), SEQ ID NO: 4 (D3r), SEQ ID NO: 5 (D3p), SEQ ID NO: 6 (D3a) und/oder Homologe, Fragmente und Teile davon. Diese Peptide sind Substanz-Einheiten (im Folgenden oft "Monomere" genannt), die an Amyloid-Beta-Spezies binden.

In einer Variante der Erfindung werden solche Monomere eingesetzt, die an die Bestandteile von Amyloid-Fibrillen, z.B. SEVI oder Aβ, mit einer Dissoziationskonstante (K_{D}-Wert) von höchstens 500 µM, bevorzugt 250, 100, 50 µM, besonders bevorzugt 25, 10, 6 µM, insbesondere 4, 2, 1 µM bindet.

Offenbart werden Polymere, die aus zwei oder mehreren der oben genannten Monomere bestehen, insbesondere Dimere der SEQ ID NO: 7 (RD2D3), SEQ ID NO: 8 (D3RD2), SEQ ID NO: 9 (D3D3), SEQ ID NO: 10 (RD2RD2) und / oder SEQ ID NO: 11 (DB3DB3) und/oder deren Homologe, Fragmente und Teile davon. Die Dimere sind aus zwei Monomer-Einheiten aufgebaut, die an Amyloid-Beta-Spezies und / oder SEVI assoziierte Fibrillen binden.

Die erfindungsgemäß aufgebauten Polymere aus Monomeren, die ihrerseits an amyloide Fibrillen wie z. B. der Alzheimerschen Demenz bzw. z. B. der SEVI binden bzw. deren Bildung verhindern oder diese zerstören, zeigen deutliche, synergetische Effekte in Bezug auf ihre Selektivität und Affinität im Vergleich zu den Monomeren. Mit anderen Worten: Die erfindungsgemäßen Polymere, insbesondere die Dimere ausgewählt aus der Gruppe von SEQ ID NO:7, SEQ ID NO: 8, SEQ ID NO: 9, SEQ ID NO: 10 und /oder SEQ ID NO: 11 sind den Monomeren, aus denen sie aufgebaut sind, überlegen. Synergetische Effekte im Sinne der vorliegenden Erfindung sind Effekte, die eine höhere Selektivität und / oder Affinität bezüglich der Bestandteile von Amyloid-Fibrillen, z.B. SEVI oder Aβ, zeigen, insbesondere des K_{D}-Wertes betreffend die Bestandteile von Amyloid-Fibrillen, z.B. SEVI oder Aβ, im Vergleich zu den einzelnen Monomer-Einheiten.

In einer weiteren besonders vorteilhaften Ausgestaltung der Erfindung wirken die Polymere und insbesondere Dimere im Tiermodellversuch (*in vivo*) vorteilhaft effizienter als die Monomere.

In einer Variante der Erfindung werden solche Polymere eingesetzt, die an amyloide Fibrillen, z. B. des A-Beta-Peptids mit einer Dissoziationskonstante (K_{D}-Wert) von höchstens 500 µM, bevorzugt 250, 100, 50 µM, besonders bevorzugt 25, 10, 1 µM, besonders bevorzugt mit einer Dissoziationskonstante (K_{D}-Wert) von höchstens 500 nM, 250, 100, 50, besonders bevorzugt 25, 10, 1 nM, 500 pM, 100, 50, 25, 10, 9, 8, 7, 6, 5, 4, 3, 2, 1 pM bis sub-pM binden.

In einer Ausgestaltung der Erfindung ist die Affinität über die Dissoziationskonstante (K_{D}-Wert) definiert.

Offenbart ist, dass Fragmente und Teile eine ähnliche beziehungsweise identische Wirkung zeigen wie die erfindungsgemäßen Peptide.

In einer Variante bestehen die offenbarungsgemäßen Peptide, insbesondere die Peptide gemäß der SEQ ID NO: 1, SEQ ID NO: 2, SEQ ID NO: 3, SEQ ID NO: 4, SEQ ID NO: 5, SEQ ID NO: 6, SEQ ID NO: 7 und / oder SEQ ID NO: 8, SEQ ID NO: 9, SEQ ID NO: 10 und / oder SEQ ID NO: 11 und deren Homologe, Fragmente oder Teile davon im Wesentlichen, bevorzugt mindestens 50%, 60%, 75%, 80%, besonders bevorzugt 85%, 90%, 95%, insbesondere 96%, 97%, 98%, 99%, 100% aus D-Aminosäuren.

Ein Polymer im Sinne der Offenbarung ist gebildet aus 2, 3, 4, 5, 6, 7, 8, 9, 10, 11, 12, 13, 14, 15, 16, 17, 18, 19, 20 oder mehr Monomeren, die an die Bestandteile von Amyloid-Fibrillen, z.B. SEVI oder Aβ, binden.

Das Polymer ist insbesondere aus Monomeren gebildet ausgewählt aus der Gruppe bestehend aus SEQ ID NO: 1, SEQ ID NO: 2 und /oder SEQ ID NO: 3.

Das Polymer kann in einer Ausgestaltung der Offenbarung ausgewählt sein aus der Gruppe der SEQ ID NO: 7, SEQ ID NO: 8, SEQ ID NO: 9, SEQ ID NO: 10 und / oder SEQ ID NO: 11 und deren Homologe, Fragemente oder Teile davon, die für sich bereits an Amyloid-Beta-Oligomer der Alzheimerschen Demenz binden.

In einer weiteren Ausgestaltung der Erfindung umfassen erfindungsgemäße Peptide mindestens ein an ein Amyloid-Beta-Spezies bindendes Peptid, wie z. B. das aus dem Stand der Technik bekannte D3, wobei das Peptid eine lineare Aminosäuresequenz aufweist, die es ihn dazu befähigen, an A-Beta zu binden, und diese Eigenschaft entweder erhalten bleibt oder verstärkt wird, in dem das lineare Peptid durch kovalente Verknüpfung seiner beiden Enden letztlich in zyklisierter Form vorliegt.

Das genannte Peptid D3 ist z. B. aus der WO 02/081505 A2 bekannt.

Das erfindungsgemäße Peptid weist vorteilhaft eine Aminosäuresequenz auf, bei der die Zyklisierung des linearen Moleküls, das heißt der ersten mit der letzten Aminosäure, durch eine kovalente Bindung, z.B. durch eine Kondensationsreaktion, erfolgt.

Diese Maßnahme bewirkt vorteilhaft, dass keine offenen Enden in der Peptidkette (Aminosäuresequenz) vorliegen.

Diese Maßnahme bewirkt ferner, dass alle linearen Peptide mit Aminosäuresequenzen, die nach der Zyklisierung die gleiche, nicht mehr unterscheidbare Aminosäure-Reihenfolge ergeben, identisch sind.

Beispiel: Die lineare Aminosäuresequenz von D3 ist rprtrlhthrnr. Das entsprechende durch eine Amidbindung zwischen der N-terminalen Aminogruppe und der C-terminalen Carboxylgruppe verknüpfte, zirkularisierte Peptid "cD3" ist nicht mehr zu unterscheiden von den zirkularisierten Peptiden prtrlhthrnrr, rtrlhthrnrrp, trlhthrnrrpr, rlhthrnrrprt, lhthrnrrprtr, hthrnrrprtrl, thrnrrprtrlh, hrnrrprtrlht, rnrrprtrlhth, nrrprtrlhthr, oder rrprtrlhthrn.

Die Herstellung zyklisierter Peptide ist Stand der Technik, und kann beispielweise nach den Verfahren wie beschrieben in DE 102005049537 A1 oder http://www.beuth-hochschule.de/fileadmin/oe/tt/Forschungsassistenz/ Forschungsassistenz 01/tt fa 01 schuster.pdf ("Synthese von zyklischen Hexapeptiden mit Blutdrucksenkender Wirkung") erfolgen.

Vorteilhaft bewirkt auch die Zyklisierung, dass keine "offenen" Enden der Peptidkette mehr existieren, die oft Angriffspunkte für Peptid-abbauende Aktivitäten in Zellen, Tieren oder Menschen sind, z. B. durch Aminopeptidasen und Carboxypeptidasen. Zyklisierte Peptide haben also vorteilhaft eine weit größere Stabilität in Tier und Mensch als die entsprechenden linearen Peptide.

Das zyklisierte Peptid kann eine Linker-Gruppe aufweisen. Mit dem Begriff Linker-Gruppe sind vorzugsweise gemeint: zusätzliche einzelne Aminosäuren in Molekülen wie z. B. D3, die per se bei der Behandlung der Alzheimerschen Demenz eingesetzt werden können, um A-Beta-Bestandteile zu binden. Durch die zusätzliche Aminosäure soll die Bindung des zyklisierten Peptids an A-Beta nicht beeinträchtigt werden.

Einige lediglich beispielhaft genannte Sequenzen zu Peptiden, die erfindungsgemäß einsetzbar sind, sind nachfolgend dargestellt:
D3 rprtrlhthrnr (SEQ ID NO: 1)
RD2 ptlhthnrrrrr (SEQ ID NO: 2)
DB3 rpitrlrthqnr (SEQ ID NO: 3)
D3r rprtrlhthrnrr (SEQ ID NO: 4)
D3p rprtrlhthrnrp (SEQ ID NO: 5)
D3a rprtrlhthrnra (SEQ ID NO: 6)
RD2D3 ptlhthnrrrrrrprtrlhthrnr (SEQ ID NO: 7)
D3RD2 rprtrlhthrnrptlhthnrrrrr (SEQ ID NO: 8)
D3D3 rprtrlhthrnrrprtrlhthrnr (SEQ ID NO: 9)
RD2RD2 ptlhthnrrrrrptlhthnrrrrr (SEQ ID NO: 10)
DB3DB3 rpitrlrthqnrrpitrlrthqnr (SEQ ID NO: 11)

Die Monomeren Peptid-Bausteine können identisch oder nicht-identisch sein.

Die Peptid-Bausteine können Kopf an Kopf, Schwanz an Schwanz oder Kopf an Schwanz linear gekoppelt und insgesamt durch kovalente Verknüpfung, mit oder ohne zusätzliche Linker-Bausteine (z. B. ein oder mehrere Aminosäuren), der beiden verbleibenden Enden zyklisiert oder amidiert oder mit anderen Gruppen besetzt am C-terminus vorliegen.

Die mindestens zwei Peptid-Monomer-Einheiten, können dabei wiederum kovalent oder nicht-kovalent, z. B. über eine Biotin-Gruppe und einen Streptavdin-Tetramer miteinander verknüpft sein. Die Peptide sind durch Einheiten gekennzeichnet, welche Kopf an Kopf, Schwanz an Schwanz oder Kopf an Schwanz linear gekoppelt sind.

Da das Zielmolekül der therapeutischen Behandlung z. B. amyloide Fibrillen der Alzheimerschen Demenz und / oder SEVI assoziierte Fibrillen oder ein Amyloid-Beta-Oligomer und damit natürlicherweise ein multivalentes Target ist, werden in einer besonders bevorzugten Ausgestaltung der Erfindung Substanzen eingesetzt, die für eine Behandlung, das heißt Zerstörung vorhandener Amyloid-Beta-Oligomere und insbesondere amyloide Fibrillen der Alzheimerschen Demenz und / oder SEVI assoziierte Fibrillen, die aus mehreren Kopien einer bereits effizient Amyloid-Beta-Oligomer-bindenden Einheit oder aus mehreren verschiedenen bereits effizient Amyloid-Beta-Oligomer-bindenden Einheiten besteht.

Diese Peptide können zyklisiert vorliegen. In diesem Fall umfasst das erfindungsgemäße Peptid also mehrere, an sich wirksam an Amyloid-Beta-Oligomer bindende Peptid-Bausteine, welche jeweils aus Aminosäuren aufgebaut und insgesamt durch kovalente Verknüpfung zyklisiert ist.

Insbesondere sind alle linearen Peptide mit Aminosäuresequenzen, die nach der Zyklisierung die gleiche, nicht mehr unterscheidbare Aminosäure-Reihenfolge ergeben, identisch. Beispiel: Die lineare Aminosäuresequenz von D3 ist rprtrlhthrnr. Das entsprechende durch eine Amidbindung zwischen der N-terminalen Aminogruppe und der C-terminalen Carboxylgruppe verknüpfte, zirkularisierte Peptid "cD3" ist nicht mehr zu unterscheiden von den zirkularisierten Peptiden prtrlhthrnrr, rtrlhthrnrrp, trlhthrnrrpr, rlhthrnrrprt, Ihthrnrrprtr, hthrnrrprtrl, thrnrrprtrlh, hrnrrprtrlht, rnrrprtrlhth, nrrprtrlhthr, oder rrprtrlhthrn.

In einer Variante der Offenbarung handelt es sich um ein Agenz mit einem Peptid mit der Aminosäuresequenz gemäß SEQ ID NO: 1 und / oder der SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 und / oder Homologe davon mit einer Identität von 50 %. "Homologe Sequenzen" oder "Homologe" bedeutet im Sinne der Offenbarung, dass eine Aminosäuresequenz eine Identität mit einer der oben genannten Aminosäuresequenz der Monomere von mindestens 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 % aufweist. Anstelle des Begriffs "Identität" werden in der vorliegenden Beschreibung die Begriffe "homolog" oder "Homologie" gleichbedeutend verwendet. Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl, Math. 2: 482-489 (1981)) berechnet unter Einstellung folgender Parameter für Aminosäuren: Gap creation penalty: 8 und Gap extension penalty: 2; und folgender Parameter für Nukleinsäuren: Gap creation penalty: 50 und Gap extension penalty: 3. Bevorzugt wird die Identität zwischen zwei Nukleinsäuresequenzen oder Polypeptidsequenzen durch die Identität der Nukleinsäuresequenz / Polypeptidsequenz über die jeweils gesamte Sequenzlänge definiert, wie sie durch Vergleich mit Hilfe des Programms GAP basierend auf dem Algorithmus von Needleman, S.B. und Wunsch, CD. (J. Mol. Biol, 48: 443-453) unter Einstellung folgender Parameter für Aminosäuren berechnet wird: Gap creation penalty: 8 und Gap extension penalty: 2; und die folgenden Parameter für Nukleinsäuren Gap creation penalty: 50 und Gap extension penalty: 3.

Zwei Aminosäuresequenzen sind im Sinne der vorliegenden Erfindung identisch wenn sie dieselbe Aminosäuresequenz besitzen. Unter Homologe sind in einer Variante die entsprechenden retro-inversen Sequenzen der oben genannten Monomere zu verstehen. Mit dem Begriff "retro-inverse Sequenz" wird erfindungsgemäß eine Aminosäuresequenz bezeichnet, die sich aus Aminosäuren in der enantiomeren Form zusammensetzt (invers: Chiralität des alpha-C-Atoms invertiert) und bei der zusätzlich die Sequenzreihenfolge zur ursprünglichen Aminosäuresequenz umgekehrt wurde (retro = rückwärts).

In einer weiteren Variante binden die erfindungsgemäßen Peptide auch an Teile des Amyloid Beta-Peptids.

In einer weiteren Variante weisen die Agenzien Peptide und Sequenzen auf, die sich von den angegebenen Sequenzen um bis zu drei Aminosäuren unterscheiden.

Ferner werden Peptide mit Sequenzen eingesetzt, die die oben genannten Sequenzen enthalten.

In einer weiteren Variante weisen die Peptide Fragmente der oben genannten Sequenzen auf oder weisen homologe Sequenzen zu den oben genannten Sequenzen auf.

Fragmente und Teile zeigen eine ähnliche beziehungsweise identische Wirkung wie die offenbarungsgemäßen Peptide.

Die offenbarungsgemäßen Agenzien werden zur Behandlung und / oder Prävention von HIV und / oder anderer viraler Infektionen eingesetzt.

Polymere von erfindungsgemäßen Peptiden mit den genannten Aminosäuresequenzen, die zur Behandlung und / oder Prävention der Alzheimerschen Demenz assoziierten Fibrillen geeignet sind lösen die Aufgabe der Erfindung ebenfalls. Diese werden offenbarungsgemäßen zur Behandlung und / oder Prävention von HIV und / oder anderer viraler Erkrankungen eingesetzt. Dies sind Agenzien, enthaltend mindestens eine Substanz, die dazu geeignet ist, die Bildung von amyloiden Fibrillen wie z. B.

Aβ-Fibrillen der Alzheimerschen Demenz zu inhibieren oder diese zu zerstören. Diese werden zur Behandlung und / oder Prävention von HIV und / oder anderer viraler Infektionen sowie Folgeerkrankungen, wie z. B. HAD, eigesetzt, indem amyloide Fibrillen, die die virale Infektionseffizienz erhöhen, in ihrer Bildung verhindert werden oder diese zerstört werden.

Offenbarungsgemäß werden die Agenzien als Therapeutikum und zur Prävention von HIV und anderer viraler Erkrankungen verwendet.

Das offenbarungsgemäße Agenz, eine Creme oder eine Tablette als Darreichungsform umfasst daher ein Peptid, enthaltend mindestens eine Aminosäuresequenz ausgewählt aus der Gruppe bestehend aus SEQ ID NO: 1 und / oder eine der angegebenen SEQ ID NO: 2-11 oder Homologe, oder Fragmente und Teile davon sowie Polymere und deren Homologe, Fragmente und Teile davon. Andere Applikationsarten, z.B. subkutan, intraperitoneal, intracranial, in Form von Zäpfchen, als Nasenspray, und so weiter sind erfindungsgemäß ebenfalls umfasst. Dann umfassen diese Darreichungsformen die erfindungsgemäßen Agenzien.

Weitere Peptid-Sequenzen, die zur Behandlung und Prävention der Alzheimerschen Demenz nachweislich verwendet werden können, sind auch für die Prävention von HIV und anderer viraler Infektionen oder deren Ausbreitung im Körper nutzbar und daher Gegenstand der vorliegenden Offenbarung, da diese als Zieltarget nicht nur die amyloiden Fibrillen der Alzheimerschen Demenz sondern gegebenenfalls auch der SEVI assoziierte Fibrillen haben.

Im Rahmen der Erfindung wurde erkannt, dass für die Verhinderung einer Infektion z. B., aber nicht ausschließlich, SEVI-Fibrillen in Sperma als Zielmoleküle in Betracht kommen. Bei bereits Infizierten kann erfindungsgemäß insbesondere, aber nicht ausschließlich, die Entstehung der HAD verhindert werden oder deren Progression verlangsamt werden, in dem die Zahl der im ZNS mit zunehmendem Lebensalter immer häufiger vorkommenden Amyloid-Fibrillen (bestehend z.B. aus Aβ) reduziert wird und so die Infektionseffizienz von HIV möglichst stark verringert wird.

Im Rahmen der Erfindung wurde weiterhin erkannt, dass die erfindungsgemäßen Peptide als Bestandteile der erfindungsgemäßen Agenzien die Erhöhung der Effizienz einer HIV-Infektion durch Amyloid-Fibrillen, bestehend z.B. aus SEVI oder Aβ reduzieren und damit die Ansteckung durch sexuelle Transmission verhindern und / oder die Ausbreitung des Virus im Körper verlangsamen. Mit "Aβ" sind alle Spezies von humanem Aß und den im Menschen beschriebenen und bekannten Varianten und Mutanten gemeint, zum Beispiel Aβ₁₋₄₀, Aβ₁₋₄₂, Aβ₃₋₄₀, Aβ_{pE3-40}, Aβ_{pE3-42}, sowie Mischungen daraus. Erfindungsgemäße Agenzien verhindern die Bildung oder zerstören mindestens eine der genannten, infektionssteigernden amyloiden Fibrillen.

Im Rahmen der Erfindung wurde erkannt, dass nur die Aβ-Fibrillen nicht aber die Aβ-Monomere oder die Aβ-Oligomere die HIV-1 Infektion erhöhen können. Gegenstand der Erfindung sind daher insbesondere diejenigen erfindungsgemäßen Peptide, als Bestandteile der Agenzien, Cremes, Tabletten, Zäpfchen, Sprays und so weiter, die zur Behandlung und zur Prävention der Alzheimerschen Demenz nachweislich verwendet werden, indem sie z. B. die Aβ-Fibrillen binden und / oder zerstören. Diese Peptide als Bestandteil der Agenzien, Cremes, Tabletten, Injektions- und Infusionslösungen und so weiter, sind besonders auch für die Prävention von HIV und anderer viraler Infektionen nutzbar und daher Gegenstand der vorliegenden Offenbarung.

Es wurde ferner erkannt, dass Peptide, wie beispielweise das Peptid D3 und das Peptid D3D3 gemäß der SEQ ID NO: 1, bzw. der SEQ ID NO: 9, die unter anderem die aus Aβ₁₋₄₂ aufgebauten Fibrillen der Alzheimerschen Demenz besonders wirksam eliminieren, auch amyloide Sperma assoziierte Fibrillen als Träger und Enhancer für Viren (SEVI), insbesondere Retroviren, wie z. B. HIV wirksam eliminieren. Es wurde überraschend erkannt, dass trotz der unterschiedlichen Zusammensetzung der körpereigenen Fibrillen, diese offensichtlich derart ähnliche Strukturen ausbilden, dass diese mittels eines gemeinsamen Wirkstoffs, wie z. B. D3 nach SEQ ID NO: 1 bzw. D3D3 nach SEQ ID NO: 9, bzw. der übrigen genannten Agenzien besonders wirksam eliminiert werden können.

Die Offenbarung ist hierauf nicht beschränkt. Die erfindungsgemäßen Agenzien und Peptide können auch andere virale Infektionen und deren Folgeerkrankungen, wie z. B. die HAD wirksam verhindern.

Die Offenbarung und die Nutzung der Agenzien beziehen sich daher nicht nur auf Retroviren, sondern auch auf Rhabdoviren, wie z. B. das humanpathogene Vesikular Stomatitis-Virus.

Die Verwendung der erfindungsgemäßen Peptide als Therapeutikum und zur Prävention von beispielweise HIV und anderer, oben genannter Viren ist daher unmittelbarer Gegenstand der Offenbarung.

Insbesondere die Verwendung der erfindungsgemäßen Peptide als Therapeutikum oder Präventionsmittel für die Verhinderung der Bildung bzw. Zerstörung von vorhandenen Amyloid-Fibrillen der Alzheimerschen Demenz (Aβ-Fibrillen) und SEVI-Fibrillen, um deren HIV-Infektionseffizienz-erhöhende Wirkung zu inhibieren ist unmittelbarer Gegenstand der Offenbarung.

Es existieren Kreuzeinwirkungen amyloider Fibrillen. Amyloide Fibrillen der Alzheimerschen Demenz und / oder der SEVI assoziierten Fibrillen aber auch Fibrillen, die aus anderen Proteinen/Peptiden bestehen, können gleichzeitig mit erfindungsgemäßen Agenzien behandelt werden. Agenzien, die gegen die amyloiden Fibrillen der einen Krankheit verwendet werden können auch gegen eine andere, gegebenenfalls amyloide Fibrillen ausbildende Krankheit, verwendet werden.

Mit Hilfe unterschiedlicher Phagendisplaytechniken können solche Peptide identifiziert werden, die zum Beispiel mit hoher Bindungsaffinität an das Alzheimerassoziierte Aß-Peptid oder andere Fibrillen-bildende Peptide binden. Diese selektierten Peptide werden sodann als Bestandteile offenbarungsgemäßer Agenzien eingesetzt.

Einfacher für den Fachmann ist der Weg einer Literaturstudie als Verfahren, die er ohne einen besonderen Aufwand betreiben kann. Hierzu kann er die Literatur nach Wirkstoffen durchkämmen, die gegen amyloide Fibrillen einer ersten Krankheit, beispielweise der Alzheimerschen Demenz eingesetzt werden. Er ermittelt ohne Aufwand einen potentiellen Wirkstoff A. Dieser ist nicht nur gegen die erste Krankheit sondern offenbarungsgemäß einsetzbar zur Therapie und / oder Prävention einer zweiten Viruserkrankung, die durch dieselben oder weitere amyloide Fibrillen gesteigert wird, oder die ebenfalls amyloide Fibrillen ausbildet, z. B. Virusinfektionen, welche SEVI assoziiert sind.

Eine offenbarungsgemäße Verwendung liegt somit darin, ein bekanntes Agenz (z. B. D3 oder ein Peptid nach SEQ ID NO: 1-11), das gegen eine erste Krankheit, in deren Verlauf eine erste Sorte amyloide Fibrillen ausgebildet wird (Alzheimerschen Demenz) wirksam zur Therapie und / oder Prävention eingesetzt werden kann, zur Therapie und / oder Prävention einer zweiten Krankheit eingesetzt wird, die durch amyloide Fibrillen der ersten oder einer weiteren Sorte gesteigert wird.

Im Vergleich zu L-enantiomeren Peptiden sind D-Peptide *in vivo* resistenter gegenüber Proteasen und lösen, wenn überhaupt, eine schwache Immunantwort hervor.

In einer Variante bestehen die Peptide gemäß der SEQ ID NO: 1-11 und deren Homologe daher im Wesentlichen, bevorzugt mindestens zu 60 %, 75 %, 80 %, besonders bevorzugt zu 85 %, 90 %, 95 %, insbesondere zu 96 %, 97 %, 98 %, 99 %, oder ganz, das heißt zu 100 % aus D-Aminosäuren.

Die D-enantiomere Form dieser erfindungsgemäßen Peptide ist daher im Wesentlichen oder ganz in den erfindungsgemäßen Agenzien enthalten.

Offenbarungsgemäße Agenzien sind als Bestandteile z. B. in Tablettenform oder in einem mikrobioziden Gel oder Spray oder Zäpfchen oder einer anderen Darreichungsform enthalten, um eine HIV-Infektion zu therapieren bzw. zu verhindern.

Die Agenzien sind insbesondere Bestandteil eines mikrobioziden Gels. Die Aufgabe wird daher auch gelöst durch ein Agenz als Bestandteil eines mikrobioziden Gels, das geeignet ist, die Bildung von amyloiden Fibrillen zu verhindern oder diese zu zerstören.

Das mikrobiozide Gel umfasst hierzu ein Agenz, enthaltend mindestens ein Peptid mit einer Aminosäuresequenz, das zur Prävention der Alzheimerschen Demenz entwickelt bzw. erfunden wurde, in dem die damit assoziierten Amyloid-Fibrillen durch Peptide mit dieser Sequenz in ihrer Bildung verhindert oder zerstört werden. Dies können auch homologe Sequenzen, Fragmente und Teile davon sein. Das mikrobiozide Gel wird zur Behandlung und / oder Prävention von HIV und / oder anderer viraler Infektionen verwendet.

Insbesondere ist ein Agenz als Bestandteil eines mikrobioziden Gels zur Behandlung und Prävention der HIV und anderer viraler Infektionen geeignet, das ein Peptid mit mindestens einer Aminosäuresequenz gemäß der SEQ ID NO: 1-6 und Homologe, Fragmente und Teile davon und / oder Polymere der SEQ ID NO: 7-11 und deren Homologe, Fragmente und Teile davon umfasst.

Die Konzentrationsangaben der Bestandteile und insbesondere der erfindungsgemäßen Peptide im Gel werden vom Fachmann in geeigneter Weise festgelegt, ebenso die weiteren Bestandteile im Gel, wie Gleitmittel zur Anwendung im Intimbereich und so weiter.

Selbiges gilt für die Zusammensetzung einer Tablette zur Therapie von HIV oder zur Therapie und/oder Prävention der HAD. Die Agenzien sind insbesondere Bestandteil einer Tablette. Die Aufgabe der Erfindung wird daher auch gelöst durch ein Agenz als Bestandteil einer Tablette, das geeignet ist, die Bildung von amyloiden Fibrillen zu verhindern oder diese zu zerstören.

Die Tablette umfasst hierzu ein Agenz, enthaltend mindestens ein Peptid mit einer Aminosäuresequenz, das zur Prävention der Alzheimerschen Demenz entwickelt bzw. erfunden wurde, in dem die damit assoziierten Amyloid-Fibrillen durch Peptide mit dieser Sequenz in ihrer Bildung verhindert oder zerstört werden. Dies können auch homologe Sequenzen, Fragmente und Teile davon sein. Die Tablette wird zur Behandlung und / oder Prävention von HIV und / oder anderer viraler Infektionen und deren Folgeerkrankungen wie HAD verwendet.

Insbesondere ist ein Agenz als Bestandteil einer Tablette zur Behandlung und Prävention der HIV- und anderer viraler Infektionen, aber auch von Folge-Erkrankungen, wie z.B. der HAD, geeignet, das ein Peptid mit mindestens einer Aminosäuresequenz gemäß der Monomere der SEQ ID NO: 1-6 und Homologe, Fragmente und Teile davon und / oder Polymere wie z. B. die Polymere der SEQ ID NO: 7-11 und deren Homologe, Fragmente und Teile davon umfasst.

Die Konzentrationsangaben der Bestandteile und insbesondere der erfindungsgemäßen Peptide in der Tablette werden vom Fachmann in geeigneter Weise festgelegt, ebenso die weiteren Bestandteile der Tablette.

Wie für das Gel bzw. die Tablette angegeben, werden auch andere genannte Darreichungsformen wie z. B. ein Spray oder eine Injektions- oder Infusionslösung oder ein Zäpfchen die erfindungsgemäßen Agenzien enthalten.

Besonders vorteilhaft kann das erfindungsgemäße Peptid, z. B. D3 nach SEQ ID NO: 1 und das Polymer, z. B. D3D3 nach SEQ ID NO: 9 gleichzeitig die Aβ-Fibrillen als auch die SEVI-Fibrillen vermittelte HIV-Infektionssteigerung effektiv bzw. vollständig verhindern.

Im Rahmen der Erfindung wurde erkannt, dass Befunde aus dem Stand der Technik, insbesondere aus Wojtowicz et al. von rein theoretischer Bedeutung sind, da ein Zusammentreffen von Aβ-Fibrillen, HIV-Partikeln und CD4-positiven Zellen alles andere als nahe liegt und unter normalen Bedingungen im Körper als ausgeschlossen galt (Blut-Hirn-Schranke).

Wie bereits erwähnt, hat sich allerdings in den vergangenen Jahren die Prognose HIV-infizierter Patienten durch die die heute verfügbare Therapie (HAART) erheblich verbessert. Die Therapie ist lebenslang erforderlich, da die virale Infektion in der Regel nur unterdrückt, nicht aber ausgeheilt wird. Es wurde im Rahmen der Erfindung also erkannt, dass viele der HIV-positiven Patienten zwei bis drei Jahrzehnte nach Infektion neurologische Defizite entwickeln, die man unter anderem unter dem Begriff "HIV-assoziierte Demenz" (HAD) zusammenfasst. Es wurde erkannt, dass durch die hohe Zahl der HIV-positiven Langzeitüberlebenden die HAD mittlerweile die häufigste Demenzform bei unter 50-jährigen Menschen ist. Die Entstehung der HAD ist dabei völlig ungeklärt. Man geht davon aus, dass ein Überwinden der Blut-Hirn-Schranke durch HIV ein extrem seltenes Ereignis sein sollte. Insbesondere muss man davon ausgehen, dass HIV in den Zellen des zentralen Nervensystems (ZNS) wenn überhaupt, nur sehr ineffizient replizieren kann. Unabhängig von allen bisher zu HIV bekannten Informationen geht man heute davon aus, dass die Bildung der ersten Aβ-Fibrillen bereits einige Jahrzehnte vor der Entstehung der ersten klinisch beobachtbaren Symptome von "Alzheimer" beginnt.

Entscheidend für die vorliegende Erfindung ist die folgende Hypothese, die bisher in keiner Weise nahe gelegt war. Angenommen, dass bei einem 40-jährigen Menschen sich bereits die ersten Aβ-Fibrillen gebildet haben und im Gehirn vorliegen, so wäre es denkbar, dass einzelne HIV-Partikel, die die Blut-Hirn-Schranke überwinden, dort Aβ-Fibrillen vorfinden, die es ihnen ermöglichen, durch die vielfache Erhöhung ihrer Infektionseffizienz Zellen des ZNS zu infizieren und sich in ihnen zu vermehren und letztlich das Gehirn so schädigen, dass HAD-Symptome entstehen. Diese Hypothese wird durch die Ergebnisse der vorliegenden Patentanmeldung gestützt. Obwohl die wenigen Aβ-Fibrillen, die sich bereits im Lebensalter von ca. 40 Jahren im ZNS befinden, selbst noch keinen erkennbaren Schaden anrichten, steigt deren Zahl mit zunehmendem Lebensalter. So wäre erklärbar, dass eine HIV-Infektion trotz HAART im mittleren Lebensalter zu einer HAD führen kann. Die erfindungsgemäßen Agenzien wirken diesem Verlauf wirksam entgegen, indem sie die Bildung amyloider Fibrillen, z. B. die Aβ-Fibrillen der Alzheimerschen Demenz, verhindern oder zerstören. Die erfindungsgemäßen Agenzien schlagen dabei direkt durch auf die durch die Fibrillen ausgelöste erhöhte Infektiösität der Viruspartikel wie HIV. Die durch amyloide Fibrillen der Alzheimerschen Demenz und / oder SEVI assoziierte Fibrillen erhöhte Infektiösität kann erfindungsgemäß vollständig verhindert werden und auf das geringe, natürliche Maß der Infektiösität der Viren in Abwesenheit von Amyloid-Fibrillen zurückgesetzt werden.

In einer Ausgestaltung der Offenbarung werden daher Agenzien zur gleichzeitigen Behandlung und / oder Prävention der Alzheimerschen Demenz assoziierten Fibrillen und von HIV und / oder anderer viraler Erkrankungen sowie deren Folgeerkrankungen, wie z.B. der HAD, mit hoher Bindeaffinität verwendet. Insbesondere mit einer Bindeaffinität an Aβ-Amyloidfibrillen mit einer Dissoziationskonstante im Bereich von mikromolar, bevorzugt submikromolar, besonders bevorzugt nanomolar, ganz besonders bevorzugt von maximal 10 Picomolar und subpicomolar. Diese Voraussetzung wird z. B. durch das Peptid mit SEQ ID NO: 9, also mit D3D3 sowie anderen erfindungsgemäßen Peptiden, erreicht.

### Ausführungsbeispiele

Im Weiteren wird die Erfindung an Hand von Ausführungsbeispielen und der beigefügten Figuren näher erläutert, ohne dass dadurch der sich dem Fachmann erschließende Umfang der vorliegenden Erfindung eingeschränkt wird.

### Ausführungsbeispiel 1: Verwendung des amyloidogenen-Inhibitors D3 zur Reduzierung der SEVI- oder Aβ-vermittelten viralen Infektion

Um die Wirkung des amyloidogenen Inhibitors D3 als potenzielles Therapeutikum zur Reduzierung von Amyloid-Peptid-geförderter retroviraler Ausbreitung zu analysieren, wurden adhärent wachsende TZM-bl Zellen mit HIV-1 infiziert. Diese Zellen exprimieren die HIV-1 Rezeptoren CD4, CXCR4 und CCR5, weshalb sie sowohl für T-trope als auch M-trope HIV Isolate permissiv sind. Des Weiteren exprimieren sie die Reportergene β-Galaktosidase und Luciferase unter der Kontrolle des HIV-1 LTR Promotors, deren Expression daher durch eine HIV-1 Infektion induziert und somit nachgewiesen werden kann. Die TZM-bl Indikator-Zelllinie ermöglicht somit eine quantitative Analyse der HIV-Infektionseffizienzen.

TZM-bl-Zellen wurden mit gleichen Mengen HIV-1 infiziert, die zuvor mit verschiedenen Konzentrationen Aβ₁₋₄₂ Fibrillen präinkubiert worden sind, welche wiederum mit zwei Konzentrationen des Peptids D3 bzw. PBS als Lösungsmittelkontrolle behandelt worden waren. D3 bestand aus Aminosäuren der D-Enantiomeren Form. 48 h nach der Infektion wurden die TZM-bl Zellen mit PBS gewaschen, durch Zugabe von Formaldehyd/Glutaraldehyd fixiert und anschließend erneut gewaschen. Die β-Galaktosidase vermittelte Blaufärbung der Zellen erfolgte durch über Nacht-Inkubation mit einer X-Gal-Lösung. Am nächsten Tag wurde die Färbelösung entfernt, die fixierten Zellen erneut mit PBS gewaschen und lichtmikroskopisch auf Blaufärbung hin untersucht. Da diese Reporterzellen proportional zur Infektionsrate neben der β-Galaktosidase auch Luciferase exprimieren, konnte dieser Effekt auch quantitativ bestimmt werden. Hierzu wurden die in gleicher Weise infizierten Zellen nach zweifachem Waschen mit PBS mittels 1xPLB (passive lysis buffer) lysiert. Nach Zugabe von Luciferase Substratlösung wurde die Lichtintensität der Wellenlänge 560 nm, die durch Oxidation von Luciferin entsteht, das wiederum durch das Enzym Firefly Luciferase katalysiert wird, gemessen.

Anhand der Anzahl der blau gefärbten Zellen sowie der gemessenen Lichtintensität konnte gezeigt werden, dass D3 sowohl die durch SEVI als auch die durch Aβgeförderte Infektionsrate deutlich reduzieren konnte.

Demnach stellt D3 gemäß SEQ ID NO: 1 ein potenzielles Therapeutikum zur Eingrenzung und Prävention viraler Infektionen, insbesondere aber nicht ausschließlich von HIV sowie deren Folgeerkrankungen, wie z.B. der HAD, dar.

Neben der Rolle, die Amyloide bei der Effizienz der Infektion von HIV-Partikeln in Sperma spielen, ist anzunehmen, dass Amyloide auch eine wichtige Rolle bei der Entstehung der HIV-assoziierten Demenz (HAD) innehaben.

HAD, auch HIV-Enzephalopathie genannt, entwickelt sich bei 20 bis 30 % der HIV-positiven Patienten im Verlauf der Infektion. Es ist ein Syndrom aus kognitiven, motorischen und Verhaltens-Störungen. Dazu zählen motorische Symptome mit Störung der Feinmotorik, kognitive Symptome, z. B. Gedächtnis- und Konzentrationsstörungen, Emotionale Symptome, z.B. Verlust von Initiative und Antrieb, und sozialer Rückzug mit Verlust sozialer Kompetenz.

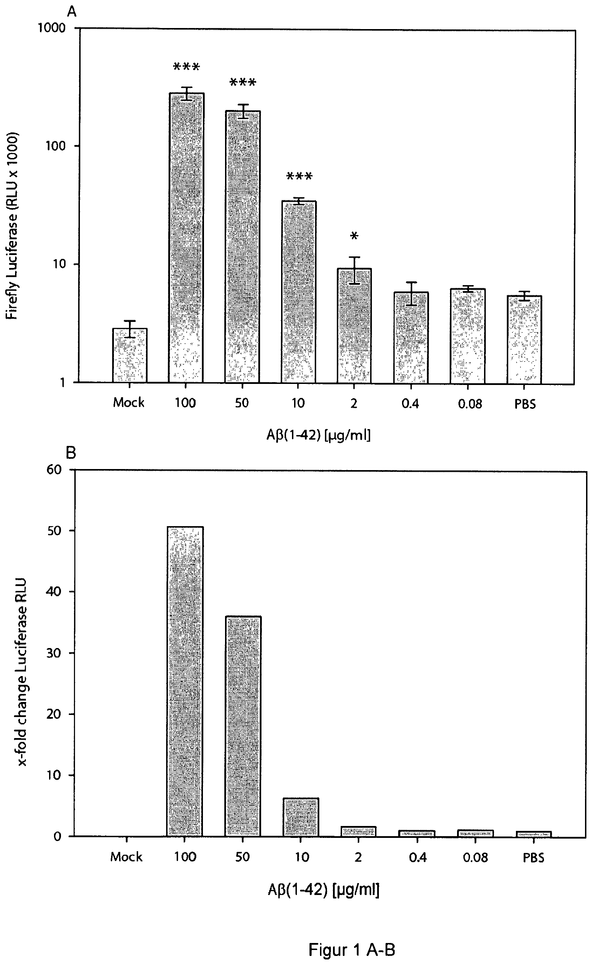

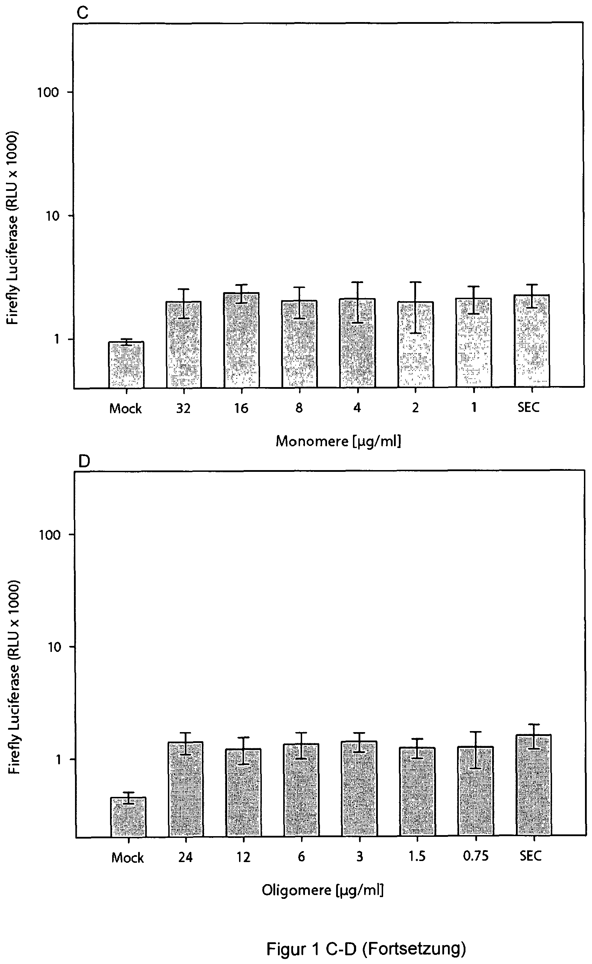

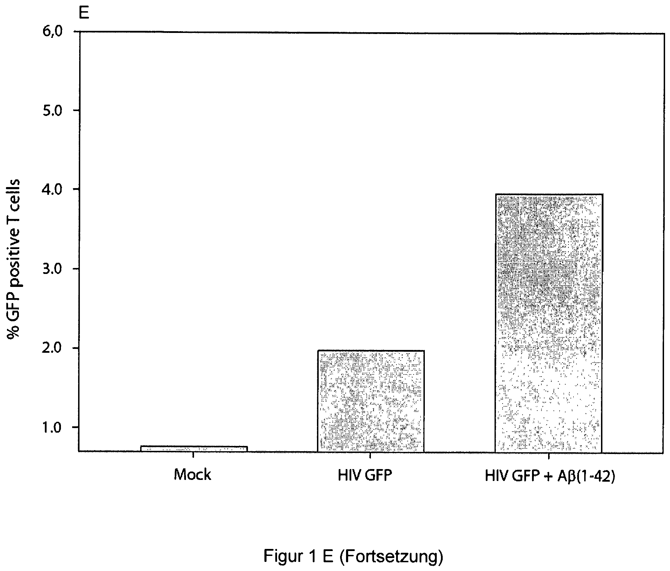

Fig. 1 zeigt, dass Alzheimersche Demenz assoziierte Aβ₁₋₄₂ Fibrillen, aber nicht Mono- und Oligomere die HIV-1 Infektion von TZM-bl Zellen erhöhen.

Gleiche Mengen von HIV-1 Isolat NL4-3 wurden für 5 min bei RT mit den angegebenen Konzentrationen von Aβ₁₋₄₂ Fibrillen vorinkubiert.

Anschließend wurden TZM-bl Reporterzellen mit vorbehandelten Viruspartikeln infiziert.

Fig. 1A zeigt die infektionsinduzierte Luciferase Aktivität 48 h nach Infektion. Fig. 1 B zeigt die Änderung der Luciferase Erhöhung relativ zu Zellen, welche in Abwesenheit der Aβ₁₋₄₂ Fibrillen (Mock-Ansatz) infiziert wurden. Fig. 1C und Figur 1D zeigen denselben Ansatz wie in Fig. 1A unter Nutzung der angegebenen Mengen an Aβ₁₋₄₂ Mono- und Oligomeren.

Figur 1E zeigt die Aβ₁₋₄₂-Fibrillen geboostete Infektiösität bei MOLT-4 T-Zellen. Gleiche Mengen an HIV-1 NL4-3 Derivaten, welche ein NEF-GFP Fusionsprotein exprimieren wurden für 5 min bei RT mit Aβ₁₋₄₂ [50 µg/ml] präinkubiert. Anschließend wurde das vorbehandelte Virus genutzt, um MOLT-4 T-Zellen zu infizieren. Dargestellt ist der prozentuale Anteil GFP-positiver Zellen angegeben durch FACS Analyse 48 h nach der Infektion.
RLU: relative light units.
SEC: Size exclusion chromatography buffer (without tween)

Hieraus abgeleitet wird die Information, dass Peptide, die wirksam eine Alzheimersche Demenz durch Verhinderung oder Prävention der assoziierten Fibrillen bedingen, auch zur Behandlung und Prävention von HIV und besonders zur Behandlung und Prävention der HAD genutzt werden können.

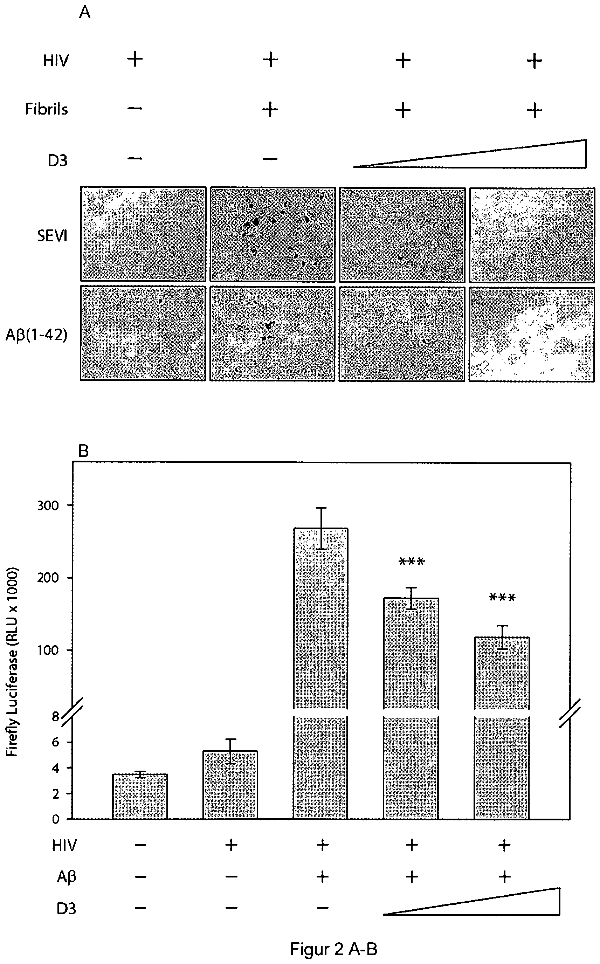

Fig. 2A zeigt einen Beweis für diese These.

Das D-Peptid D3 gemäß der SEQ ID NO: 1 wirkt als potenter amyloidogener Inhibitor. D3 reduziert die Fibrillen-getriebene Infektiösität, erkennbar an der mit zunehmender D3-Konzentration abnehmenden X-Gal Färbung der TZM-bl Reporter Zellen, welche mit HIV-1 in Beisein (Plus-Zeichen) oder in Abwesenheit von SEVI und Aβ₁₋₄₂ Fibrillen (50 µg/ml) (Minus-Zeichen) infiziert waren. Die Fibrillen waren mit D3 vor der Infektion vorinkubiert (0,5 and 5 µg/ml).

Figur 2B zeigt einen Blot, bei dem gleiche Mengen des HIV-1 Stamms NL4-3 für 5 min bei RT mit Aβ₁₋₄₂ Fibrillen (50 µg/ml) präinkubiert waren. Weiterhin wurden Fibrillen mit D3 (0,5 and 5 µg/ml) präinkubiert. Im Anschluss daran wurde das vorbehandelte Virus genutzt um TZM-bl Reporterzellen zu infizieren und die Infektionsinduzierte Luciferaseaktivität 48 h nach der Postinfektion gemessen (*** p<0.001).

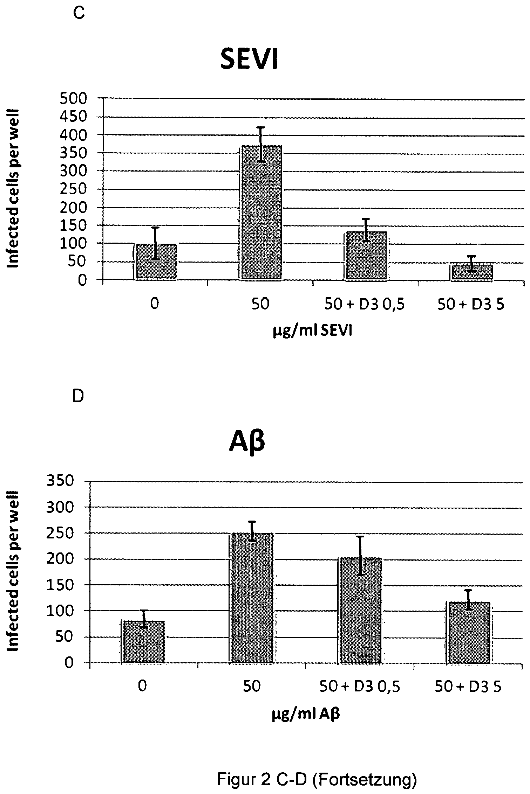

Figur 2C zeigt, dass die Anzahl infizierter Zellen mit 0,5 µg/ml D3 um etwa 60 % gegenüber den unbehandelten Zellen mit SEVI Fibrillen sinkt. Mit 5 µg/ml D3 sinkt die Anzahl der infizierten Zellen um etwa 90 % gegenüber den unbehandelten Zellen mit Amyloid-Beta-Fibrillen (Figur 2D).

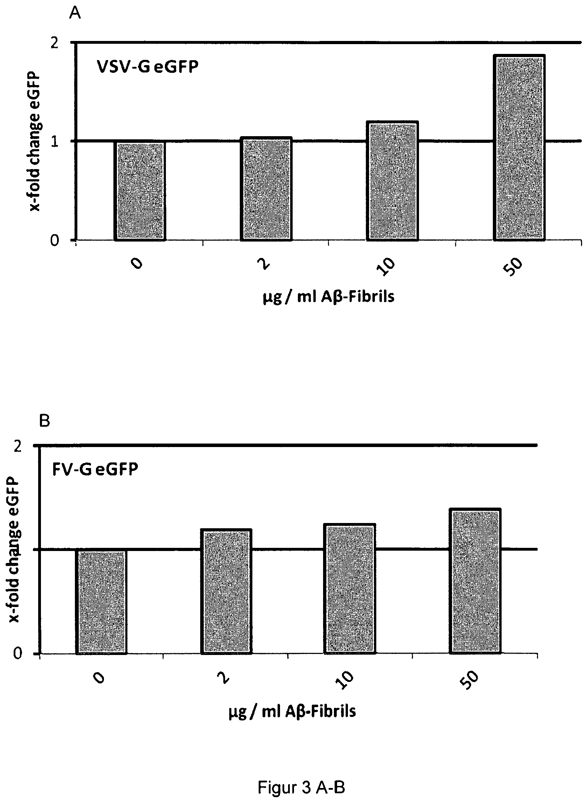

Fig. 3 zeigt, dass Aβ (1-42) Fibrillen auch in der Lage sind die Infektionsrate von pseudotypisierten HIV Partikeln zu fördern. Hierzu wurden infektiöse Viruspartikel mit Hilfe von lentiviralen Vektoren generiert, bei denen das virale Genom durch das grün fluoreszierendes Genprodukt (GFP) substituiert wurde. Für die Pseudotypisierung, das heißt Ausstattung mit HIV-fremden Glykoproteinen, wurde das G-Protein des Rhabdovirus "Vesicular Stromatitis Virus" (VSV-G) sowie das Glykoprotein des retroviralen Foamy Virus (FV-G) verwendet. Beide Glykoproteine binden an Phospholipide, die ubiquitär auf der Zellmembran vorkommen und ermöglichen dadurch ein breiteres Wirtsspektrum. Die pseudotypisierten lentiviralen Partikel wurden 5 min mit der angegebenen Konzentration von Aβ(1-42) inkubiert. Im Anschluss wurden die Viren dazu verwendet HEK293 T Zellen zu transduzieren, das heißt das Genprodukt GFP stabil in das Wirtsgenom zu integrieren. Die Anzahl der GFP positiven, also transduzierten Zellen wurde 48 h nach Transduktion mittels FACS analysiert. Abgebildet ist der x-fache Anstieg der transduzierten Zellen in Relation zu solchen, die ohne Zugabe von Aβ(1-42) transduziert worden sind.

Dieser fördernde Effekt war im Gegensatz zum HIV-1 Glykoprotein weniger effektiv, aber deutlich. Nachweislich kann die Anwesenheit von Fibrillen die Infektionsrate von Viren unabhängig von ihrer Glykoproteine bzw. unabhängig ihres Wirtspektrums fördern. Demnach eignet sich D3 auch zur Behandlung anderer viraler Erkrankungen und deren Folgeerkrankungen, wie insbesondere HAD.

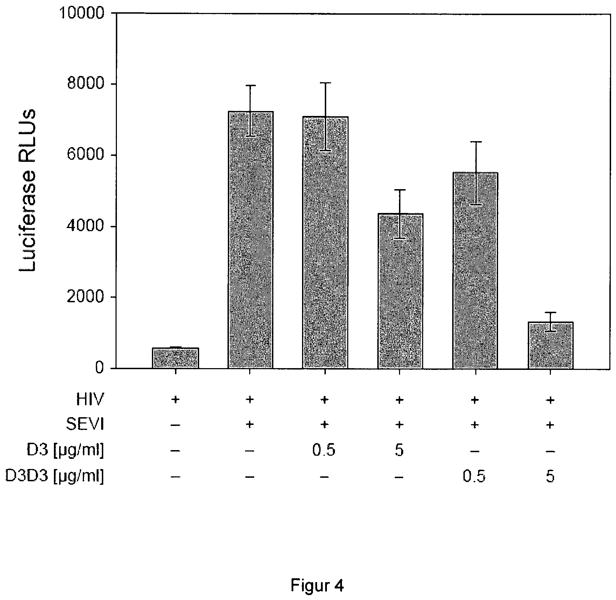

Figur 4 und die Tabelle 1 beweisen beispielhaft an Hand der Inhibitoren D3 und D3D3, dass diese auch die durch SEVI-Fibrillen erhöhte Infektiösität von HIV-1 reduzieren.

Der Versuchsansatz hierzu gliederte sich wie folgt: Die Reporterzelllinie TZM-bl wurde mit dem HIV-1 Laborstamm NL4-3 PI 952 in Anwesenheit von 6 µg/ml SEVI-Fibrillen (aus Samen isolierte, Fibrillen-bildenden PAP-Fragmente: semen derived enhancer of virus infection) inkubiert. Die Fibrillen waren mit oder ohne D3 und D3D3 (je 0,5 und 5 µg/ml) vor der Infektion vorinkubiert. Die Infektionseffizienz wurde bestimmt, in dem die Luziferaseaktivität (eingebracht durch ein im Laborstamm kodiertes Luciferase-Gen) der infizierten Zellen 48 Stunden nach der Infektion gemessen wurde (Tabelle 1: Ergebnisse zu Figur 4).

| Probenbeschreibung | Mittelwert | Standardabweichung |
|---|---|---|
| HIV + PBS | 579,0 | 21,3 |
| HIV + SEVI | 7264,0 | 704,4 |
| HIV + SEVI + 0,5 D3 | 7112,7 | 959,5 |
| HIV + SEVI + 5 D3 | 4368,0 | 676,7 |
| HIV + SEVI + 0,5 D3D3 | 5529,7 | 890,8 |
| HIV + SEVI + 5 D3D3 | 1321,0 | 252,7 |

Die Ergebnisse zeigen, dass die Peptide D3 und insbesondere D3D3, eine durch SEVI assoziierte Fibrillen gesteigerte Infektiösität der Viren um etwa 80% senken.

## Patentansprüche

1. Agenz, enthaltend mindestens eine Substanz, die dazu geeignet ist, die Bildung von amyloiden Fibrillen, die die Infektionseffizienz von HIV erhöhen, zu inhibieren oder diese zu zerstören, zur Anwendung in einem Verfahren für die Behandlung und/oder Prävention einer HIV-assoziierten Demenz (HAD), in dem amyloide Fibrillen, die die virale Infektionseffizienz erhöhen, in ihrer Bildung durch die Substanz verhindert werden oder diese zerstört werden,
**gekennzeichnet durch**
mindestens ein Peptid als Substanz mit einer Aminosäuresequenz ausgewählt aus der Gruppe bestehend aus SEQ ID NO: 1 und/oder SEQ ID NO: 2 und / oder SEQ ID NO: 3 und/oder SEQ ID NO: 4 und/oder SEQ ID NO: 5 und / oder SEQ ID NO: 6 und/oder SEQ ID NO: 7 und/oder SEQ ID NO: 8 und / oder SEQ ID NO: 9 und/oder SEQ ID NO: 10 und / oder SEQ ID NO: 11 und wobei mindestens ein Peptid im Wesentlichen oder ganz aus D-Aminosäuren besteht.

2. Agenz zur Anwendung nach einem der vorhergehenden Ansprüche, enthaltend
mindestens ein Peptid, das dazu geeignet ist, die Bildung von Amyloid-Beta-Fibrillen und SEVI-Fibrillen, die die Infektionseffizienz des HIV-Virus erhöhen, zu inhibieren oder zu zerstören zur Behandlung und / oder Prävention einer HIV-assoziierten Demenz (HAD).

3. Agenz zur Anwendung nach einem der vorhergehenden Ansprüche,
**gekennzeichnet durch**
mindestens ein Peptid als Substanz mit einer Aminosäuresequenz, das zur Prävention und Therapie der Alzheimerschen Demenz assoziierten Fibrillen geeignet ist zur Behandlung und / oder Prävention von einer HIV-assoziierten Demenz (HAD), in dem amyloide Fibrillen, die die virale Infektionseffizienz erhöhen, in ihrer Bildung durch das Peptid verhindert werden oder diese zerstört werden.

4. Agenz zur Anwendung nach einem der vorhergehenden Ansprüche,
zur gleichzeitigen Behandlung und / oder Prävention der Alzheimerschen Demenz und einer HIV-assoziierten Demenz (HAD).

5. Agenz zur Anwendung nach einem der vorhergehenden Ansprüche,
zur gleichzeitigen Behandlung und / oder Prävention der Alzheimerschen Demenz und einer HIV-assoziierten Demenz (HAD), mit einer Bindeaffinität an assoziierte Amyloidfibrillen mit einer Dissoziationskonstante im Bereich von mikromolar, bevorzugt submikromolar, besonders bevorzugt nanomolar, ganz besonders bevorzugt von maximal 10 Picomolar, besonders bevorzugt bis Subpicomolar.

6. Agenz zur Anwendung nach einem der Ansprüche 1 bis 5 als Bestandteil einer Tablette, eines mikrobioziden Gels oder Sprays oder Zäpfchens oder einer anderen Darreichungsform.

## Claims

1. Agent, comprising at least a substance, suitable to inhibit or destruct the formation of amyloid fibrilla, which enhance the infection efficiency of HIV, for the use in a process for the treatment and/or prophylaxis of HIV-associated dementia (HAD), in which amyloid fibrilla, which enhance the efficiency of viral infections, are inhibited in its formation or are destructed my means of the substance ,
**characterized by**
at least one peptide as a substance with an amino acid sequence chosen from the group consisting of SEQ ID NO: 1 and / or SEQ ID NO: 2 and / or SEQ ID NO: 3 and / or SEQ ID NO: 4 and / or SEQ ID NO: 5 and / or SEQ ID NO: 6 and / or SEQ ID NO: 7 and / or SEQ ID NO: 8 and / or SEQ ID NO: 9 and / or SEQ ID NO: 10 and / or SEQ ID NO: 11 wherein at least one peptide consists essentially or completely of D-amino acids.

2. Agent according to any one of the preceding claims,
comprising
at least one peptide, which is suitable to inhibit or destruct the formation of amyloid beta fibrilla and SEVI-fibrilla, which enhance the infection efficiency of the HIV-virus, for the treatment and/or prophylaxis of HIV-associated dementia (HAD).

3. Agent according to any one of the preceding claims,
**characterized in that**
at least one peptide as a substance with an amino acid sequence is suitable for the prophylaxis and therapy of the fibrilla associated with Alzheimer's dementia, for the treatment and/or prophylaxis of HIV-associated dementia (HAD) wherein amyloid fibrilla, which enhance the viral incektion efficiency, are inhibited in their formation my means of the peptide or are destructed.

4. Agent according to any one of the preceding claims,
for the simultaneous treatment and / or prophylaxis of Alzheimer's dementia and of HIV-associated dementia (HAD).

5. Agent according to any one of the preceding claims,
for the simultaneous treatment and / or prophylaxis of Alzheimer's dementia and of HIV-associated dementia (HAD), with a binding affinity to associated amyloid fibrilla with a dissociation constant in the range of micromolar, preferably submicromolar, particularly preferably nanomolar, in particular especially preferably of 10 picomolar at maximum, particularly preferably to subpicomolar.

6. Agent for the use of any of the claims 1 to 5 as component of a tablet, a microbiozide gel or spray or suppository or another dosage form.

## Revendications

1. Agent, comprenant au moins une substance, apte à inhiber ou détruire la formation des fibrilles amyloïdes, qui augmentent l'efficacité de l'infection par le VIH, pour l'utilisation dans une procédure pour le traitement et/ou la prévention de la démence accociée à VIH (DAV), en faisant les fibrilles amyloïdes, qui augmentent l'efficacité des infections virales, être inhibées dans leur formation ou être détruites par la substance,
**caractérisé par**
au moins un peptide comme une substance avec une séquence d'acides aminés choisie du groupe composé de SEQ ID NO: 1 et/ou SEQ ID NO: 2 et / ou SEQ ID NO: 3 et/ ou SEQ ID NO: 4 et / ou SEQ ID NO: 5 et / ou SEQ ID NO: 6 et / ou SEQ ID NO: 7 et / ou SEQ ID NO: 8 et / ou SEQ ID NO: 9 et / ou SEQ ID NO: 10 et / ou SEQ ID NO: 11 et au moins un peptide comportant essentiellement ou complètement des acides D-aminés.

2. Agent selon l'une quelconque des revendications précédentes,
comprenant
au moins un peptide, apte à inhibir ou détruire la formation des fibrilles bêta amyloïdes, qui augmentent l'efficacité de l'infection par le VIH, pour le traitement et/ou la prévention de la démence accociée à VIH (DAV).

3. Agent selon l'une quelconque des revendications précédentes,
comprenant
au moins un peptide comme une substance avec une séquence d'acide aminé, apte à la prévention et le traitement des fibrilles associées à la démence d'Alzheimer pour le traitement et/ou la prévention de la démence associée au VIH (DAV) en faisant des fibrilles amyloïdes, qui augmentent l'efficacité de l'infection virale, être inhibées par le peptide ou être détruites.

4. Agent selon l'une quelconque des revendications
pour le traitement simultané et / ou la prévention de la démence d'Alzheimer et de la démence associée au VIH (DAV).

5. Agent selon l'une quelconque des revendications précédentes
pour le traitement simultané et / ou la prévention de la démence d'Alzheimer et de la démence associée au VIH (DAV), avec une affinité de liaison aux fibrilles amyloïdes associées avec une dissociation constante dans la zone micromolaire, de préférence submicromolaire, particulièrement de préférence nanomolaire, en particulier speciellement de préférence 10 picomolaire au maximum, particulièrement jusqu'à subpicomolaire.

6. Agent pour l'utilisation selon l'une des revendications 1 à 5 comme composant d'un comprimé, d'une gel ou d'un spray ou d'une suppositoire microbiocide ou d'une autre forme d'administration.
